(19) **Europäisches Patentamt**
**European Patent Office**
**Office européen des brevets**

(11) **EP 4 715 859 A1**

(12) # EUROPEAN PATENT APPLICATION

(43) Date of publication:
**25.03.2026 Bulletin 2026/13**

(51) International Patent Classification (IPC):
*H01H 11/00* (2006.01)      *H01H 33/70* (2006.01)

(21) Application number: **24209172.6**

(22) Date of filing: **28.10.2024**

(52) Cooperative Patent Classification (CPC):
**H01H 11/00;** H01H 33/7023; Y02P 90/30

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **23.09.2024 CN 202411321424**

(71) Applicants:
• **Yunnan Power Grid Co., Ltd.
Electric Power Science Research Institute
Kunming, Yunnan Province 650000 (CN)**
• **Yunnan Electric Power Test and Research
Institute
(Group) Co., Ltd.
Kunming, Yunnan Province 650000 (CN)**

(72) Inventors:
• **WANG, Ke
Kunming, Yunnan Province (CN)**

• **LI, Xingwen
Kunming, Yunnan Province (CN)**
• **DENG, Yunkun
Kunming, Yunnan Province (CN)**
• **PENG, Jing
Kunming, Yunnan Province (CN)**
• **ZHANG, Boya
Kunming, Yunnan Province (CN)**
• **ZHAO, Xianping
Kunming, Yunnan Province (CN)**
• **CAO, Weidong
Kunming, Yunnan Province (CN)**
• **CHEN, Daoyuan
Kunming, Yunnan Province (CN)**

(74) Representative: **Meyer-Dulheuer MD Legal
Patentanwälte PartG mbB
Hanauer Landstr. 287-289
60314 Frankfurt am Main (DE)**

(54) **METHOD FOR EVALUATING NOZZLE MATERIAL OF ENVIRONMENTALLY-FRIENDLY GAS CIRCUIT BREAKER ON THE BASIS OF COMBINATION-WEIGHTED MULTI-PARAMETER DYNAMIC DECISION FACTOR**

(57)    The example of the present invention discloses a method for evaluating a nozzle material of an environmentally-friendly gas circuit breaker on the basis of a combination-weighted multiparameter dynamic decision factor. The method specifically includes: outputting a current signal; carrying out an arcing test on the nozzle material through the current signal to form an electric arc; receiving and processing a sensing electric signal of the nozzle material, and obtaining current data, voltage data, and air pressure data, where the sensing electric signal includes a current-sensing electric signal and an air pressure-sensing electric signal; detecting temperature data of the nozzle material; and evaluating the nozzle material of the environmentally-friendly gas circuit breaker through the current data, the voltage data, the air pressure data, and the temperature data of the nozzle material in the formation process for the electric arc under the actions of different output currents. According to the present invention, dynamic changes of the nozzle material and a surrounding environment of the nozzle material in the arcing process are directly reflected through the temperature data, the current data, the voltage data, and the air pressure data, so that the performance of the nozzle material can be more comprehensively and accurately evaluated.

EP 4 715 859 A1

S101

| |
|---|
| Output a current signal |

S102

| |
|---|
| Carry out an arcing test on a nozzle material through a current signal to form an electric arc |

S103

| |
|---|
| Receive and process a sensing electric signal of the nozzle material, and obtain current data, voltage data, and air pressure data, where the sensing electric signal includes a current-sensing electric signal and an air pressure-sensing electric signal |

S104

| |
|---|
| Detect temperature data of the nozzle material |

S105

| |
|---|
| Evaluate the nozzle material through the current data, the voltage data, the air pressure data, and the temperature data of the nozzle material in the formation process for the electric arc under the actions of different output currents |

FIG. 1

**Description**

**BACKGROUND OF THE INVENTION**

1. **Technical Field**

The present invention relates to the technical field of type selection and evaluation for nozzle materials of high-voltage circuit breakers, and especially relates to a method for evaluating a nozzle material of an environmentally-friendly gas circuit breaker on the basis of a combination-weighted multi-parameter dynamic decision factor.

2. **Description of Related Art**

A high-voltage circuit breaker is an important apparatus used for controlling and protecting an electric power device in an electric power system, the performance of the high-voltage circuit breaker directly influences the safety and reliability of the electric power system, and a nozzle material is one of key components in the high-voltage circuit breaker. The nozzle material plays a role of arc extinguishing in the high-voltage circuit breaker. The high-voltage circuit breaker generates an electric arc while cutting off a circuit, and a high temperature and high energy of the electric arc will cause serious damage to the nozzle material. Therefore, the nozzle material needs to have good characteristics such as high temperature resistance, high radiation resistance, and high strength to ensure stable running of the high-voltage circuit breaker. At present, an environmentally-friendly gas arc-extinguishing high-voltage circuit breaker is a hot topic of research and development, and compared with SF6 gas, mixed gases such as C4F7N have more complex erosion influence on a nozzle while being used for arc extinguishing.

In the prior art, when a state of the nozzle material is evaluated, an expensive device and a long experiment period are required, so that a test cost is increased. Meanwhile, an economic burden will also be increased due to frequent replacement for a test material. Moreover, a test environment often cannot fully simulate an actual working environment, therefore, there may be a deviation in a test result. For example, the performance under a high temperature and a high pressure may have a significant difference compared with the performance under a normal temperature and a normal pressure. Especially for evaluation for the influence of various types of new environmentally-friendly gases under arc extinguishing, there is an urgent need to propose a better evaluation method.

**BRIEF SUMMARY OF THE INVENTION**

On the basis of this, it is necessary to propose a method for evaluating a nozzle material of an environmentally-friendly gas circuit breaker on the basis of a combination-weighted multi-parameter dynamic decision factor with regard to the above problems.

The method for evaluating the nozzle material of the environmentally-friendly gas circuit breaker on the basis of the combination-weighted multi-parameter dynamic decision factor specifically includes:

outputting a current signal;
carrying out an arcing test on the nozzle material through the current signal to form an electric arc;
receiving and processing a sensing electric signal of the nozzle material, and obtaining current data, voltage data, and air pressure data, where the sensing electric signal includes a current-sensing electric signal and an air pressure-sensing electric signal;
detecting temperature data of the nozzle material; and
evaluating the nozzle material of the environmentally-friendly gas circuit breaker through the current data, the voltage data, the air pressure data, and the temperature data of the nozzle material in the formation process for the electric arc under the actions of different output currents.

The evaluating the nozzle material of the environmentally-friendly gas circuit breaker according to the current data, the voltage data, the air pressure data, and the temperature data of the nozzle material in the formation process for the electric arc specifically includes:

determining a standard voltage parameter, a standard current parameter, a standard air pressure parameter, and a standard temperature parameter according to the current data, the voltage data, the air pressure data, and the temperature data of the nozzle material under the actions of different output currents;
determining a variable voltage parameter, a variable current parameter, a variable air pressure parameter, and a variable temperature parameter according to the standard voltage parameter, the standard current parameter, the standard air pressure parameter, and the standard temperature parameter;

determining a multi-parameter dynamic decision factor according to the variable voltage parameter, the variable current parameter, the variable air pressure parameter, and the variable temperature parameter;

determining a voltage weight, a current weight, an air pressure weight, and a temperature weight according to the standard voltage parameter, the standard current parameter, the standard air pressure parameter, the standard temperature parameter, the variable voltage parameter, the variable current parameter, the variable air pressure parameter, and the variable temperature parameter;

determining the combination-weighted multi-parameter dynamic decision factor according to the voltage weight, the current weight, the air pressure weight, and the temperature weight; and

evaluating the nozzle material according to comparison conditions between the combination-weighted multi-parameter dynamic decision factor, and a qualified threshold value, a good threshold value, as well as an excellent threshold value, and classifying the performance of the nozzle material into four levels, that is, unqualified, qualified, good, and excellent.

The evaluating the nozzle material according to comparison conditions between the combination-weighted multi-parameter dynamic decision factor, and a qualified threshold value, a good threshold value, as well as an excellent threshold value, and classifying the performance of the nozzle material into four levels, that is, unqualified, qualified, good, and excellent specifically includes:

if the combination-weighted multi-parameter dynamic decision factor is determined to be less than or equal to the qualified threshold value, classifying the performance level of the nozzle material to be unqualified;

if the combination-weighted multi-parameter dynamic decision factor is determined to be greater than the qualified threshold value and less than or equal to the good threshold value, classifying the performance level of the nozzle material to be qualified;

if the combination-weighted multi-parameter dynamic decision factor is determined to be greater than the good threshold value and less than or equal to the excellent threshold value, classifying the performance level of the nozzle material to be good; and

if the combination-weighted multi-parameter dynamic decision factor is determined to be greater than the excellent threshold value, classifying the performance level of the nozzle material to be excellent.

The determining the combination-weighted multi-parameter dynamic decision factor according to the voltage weight, the current weight, the air pressure weight, and the temperature weight specifically includes:

determining the combination-weighted multi-parameter dynamic decision factor according to

$$\delta = \theta \cdot (\frac{f_1 f_2}{2} + \frac{f_1 f_3}{2} + \frac{f_1 f_4}{2} + \frac{f_2 f_3}{2} + \frac{f_3 f_4}{2})$$ , wherein $f_1$ is the voltage weight, $f_2$ is the current weight, $f_3$ is the air pressure weight, and $f_4$ is the temperature weight.

An arcing test apparatus is used for obtaining the current data, the voltage data, the air pressure data, and the temperature data of the nozzle material in the formation process for the electric arc according to claim 1, and specifically includes: a power supply assembly, a signal processing assembly, an infrared assembly, and a test assembly, where the power supply assembly is electrically connected with the test assembly, and used for outputting a current signal.

The test assembly is electrically connected with the power supply assembly, and used for carrying out an arcing test on the nozzle material through the current signal to form an electric arc.

The signal processing assembly is connected with the test assembly, and used for receiving and processing a sensing electric signal of the nozzle material, and obtaining the current data, the voltage data, and the air pressure data, where the sensing electric signal includes a current-sensing electric signal and an air pressure-sensing electric signal. the infrared assembly is connected with the nozzle material through wireless communication, and used for detecting temperature data of the nozzle material.

The test assembly includes a first electrode, a second electrode, and an arc striking metal wire,

the first electrode is connected with the second electrode through the arc striking metal wire, and the first electrode and the second electrode are used for receiving the current signal, and providing the current signal to the arc striking metal wire; and

the nozzle material is arranged at a connection section of the arc striking metal wire in a sleeved manner, one end of the nozzle material is arranged on an end part of the first electrode in a sleeved manner, the other end of the nozzle material is arranged on an end part of the second electrode in a sleeved manner, and a cavity body is formed, and used for accommodating the arc striking metal wire, and the arc striking metal wire is used for carrying out an arcing test on the nozzle material through the current signal to form an electric arc.

The test assembly further includes a first fixing clamp, a first insulation seat, a second fastening clamp, and a second insulation seat.

the first electrode is connected with the first insulation seat in a fastening manner and through the first fixing clamp, the first fixing clamp is used for fixing the first electrode, and the first insulation seat is used for supporting the fixed first electrode; and

the second electrode is connected with the second insulation seat in a fastening manner and through the second fixing clamp, the second fixing clamp is used for fixing the second electrode, and the second insulation seat is used for supporting the fixed second electrode.

The signal processing assembly includes a current mutual-induction coil, an air pressure sensor, and an integrated processor, and

the first electrode is electrically connected with a positive pole of the power supply assembly; the second electrode is electrically connected with a negative pole of the power supply assembly, and the current mutual-induction coil is arranged on a connection wire, and used for detecting a current-sensing electric signal of the nozzle material;

a penetrating hole is formed in the nozzle material on a side wall of the cavity body, and used for accommodating the air pressure sensor, and the air pressure sensor is used for detecting an air pressure-sensing electric signal of the nozzle material; and

the integrated processor is electrically connected with the test assembly, and used for receiving and processing the sensing electric signal of the nozzle material, and obtaining current data, voltage data, and air pressure data, wherein the sensing electric signal comprises the current-sensing electric signal and the air pressure-sensing electric signal.

The integrated processor includes a voltage signal port, a current signal port, and an air pressure signal port in an integrated manner,

the current mutual-induction coil is electrically connected with the current signal port, and the current signal port is used for receiving the current-sensing electric signal, and obtaining the current data through the current-sensing electric signal;

the first fixing clamp is electrically connected with a positive pole of the voltage signal port, the second fixing clamp is electrically connected with a negative pole of the voltage signal port, and the voltage signal port is used for detecting and processing a voltage-sensing electric signal of the nozzle material to obtain the voltage data; and

the air pressure signal port is electrically connected with the air pressure sensor, and the air pressure sensor is used for receiving and transmitting the air pressure data in the cavity body.

The infrared assembly receives an infrared signal of the side wall of the cavity body, which is formed by the nozzle material, and obtains the temperature data of the nozzle material through the infrared signal.

A system for evaluating a nozzle material specifically includes:

an arcing test module, used for outputting a current signal; receiving and processing a sensing electric signal of the nozzle material, and obtaining current data, voltage data, and air pressure data, where the sensing electric signal includes a current-sensing electric signal and an air pressure-sensing electric signal; detecting temperature data of the nozzle material; and carrying out an arcing test on the nozzle material through the current signal to form an electric arc.

a module for evaluating the nozzle material of the environmentally-friendly gas circuit breaker, used for evaluating the nozzle material of the environmentally-friendly gas circuit breaker through the current data, the voltage data, the air pressure data, and the temperature data of the nozzle material in the formation process for the electric arc under the actions of different output currents.

A computer-readable storage medium stores a computer program, where when the computer program is executed by a processor, the processor is enabled to execute the steps of the above method.

A computer device includes a memory and a processor, where the memory stores a computer program, and when the computer program is executed by the processor, the processor is enabled to execute the steps of the above method.

Through adopting the examples of the present invention, the following beneficial effects are achieved:

according to the present invention, the arcing test is carried out on the nozzle material of the environmentally-friendly gas circuit breaker to form the electric arc, and dynamic changes of the nozzle material and a surrounding environment of the nozzle material in the arcing process are directly reflected through the temperature data, the current data, the voltage data, and the air pressure data of the nozzle material in the formation process for the electric arc. The performance of the nozzle material can be more comprehensively and accurately evaluated through comprehensively considering weights and dynamic changes of a plurality of parameters such as an air pressure, a current, a voltage, and a temperature. The test apparatus of the method is simpler and more convenient, the test flow is clearer, the parameters considered are more comprehensive, the data processing manner is more scientific, and the performance of the material in practical application can be more effectively reflected, so that a more scientific basis is provided for selection and optimization for the material.

## BRIEF DESCRIPTION OF THE SEVERAL VIEWS OF THE DRAWINGS

In order to describe the technical solutions in the examples of the present invention or in the prior art more clearly, the drawings required to be used in the description for the examples or the prior art will be briefly introduced below, and apparently, the drawings in the description below show merely some examples of the present invention, and those of ordinary skill in the art may still derive other drawings from these drawings without creative efforts.

In the drawings:

FIG. 1 is a schematic flow diagram of an example of a method for evaluating a nozzle material of an environmentally-friendly gas circuit breaker on the basis of a combination-weighted multi-parameter dynamic decision factor, which is provided by the present invention;

FIG. 2 is a schematic structure diagram of an example of an arcing test apparatus provided by the present invention;

FIG. 3 is a schematic flow diagram of another example of a method for evaluating a nozzle material, which is provided by the present invention;

FIG. 4 is a schematic structure diagram of another example of an arcing test apparatus provided by the present invention;

FIG. 5 is an explanatory diagram of key dimensions of the arcing test apparatus provided by the present invention;

FIG. 6 is a schematic structure diagram of an example of a system for evaluating a nozzle material of an environmentally-friendly gas circuit breaker on the basis of a combination-weighted multi-parameter dynamic decision factor, which is provided by the present invention;

FIG. 7 is a schematic structure diagram of an example of a device provided by the present invention; and

FIG. 8 is a schematic structure diagram of an example of a storage medium provided by the present invention.

1. arcing test apparatus; 10. power supply assembly; 20. signal processing assembly; 30. test assembly; 40. infrared assembly; 50. nozzle material; 21. current mutual-induction coil; 22. air pressure sensor; 23. integrated processor; 231. positive port of voltage signal; 232. negative port of voltage signal; 233. current signal port; 234. air pressure signal port; 31. first electrode; 32. first fixing clamp; 33. first insulation seat; 34. second electrode, 35. second fixing clamp; 36. second insulation seat; 37. arc striking metal wire; 51. penetrating hole; 61. first cable; 62. second cable; 63. positive cable of voltage probe; 64. negative cable of voltage probe; 65. communication cable of current coil; and 66. communication cable of air pressure sensor.

## DETAILED DESCRIPTION OF THE INVENTION

The technical solutions in the examples of the present invention are clearly and completely described below in conjunction with the drawings in the examples of the present invention, and apparently, the examples described are merely a part rather than all of the examples of the present invention. On the basis of the examples of the present invention, all other examples obtained by those of ordinary skill in the art without creative efforts shall fall within the protection scope of the present invention.

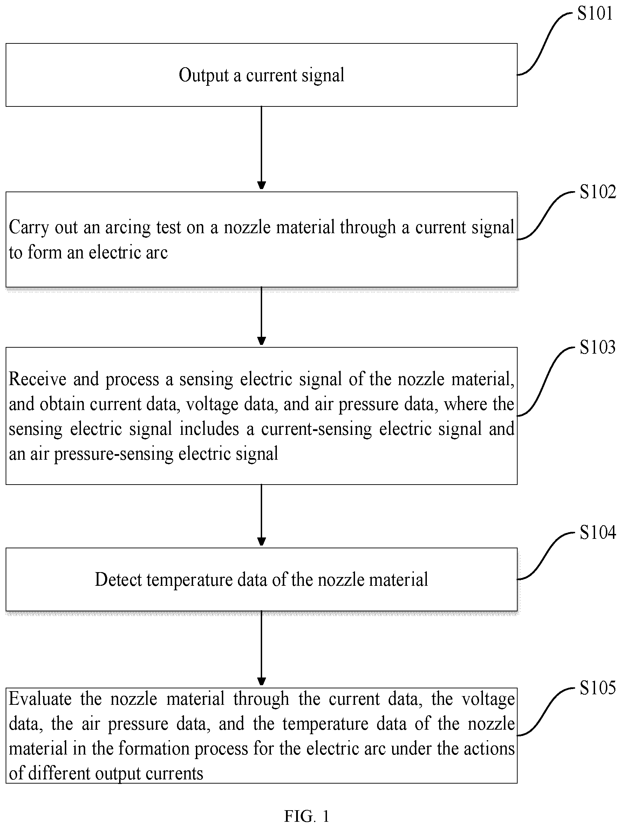

As shown in FIG. 1, FIG. 1 is a schematic flow diagram of an example of a method for evaluating a nozzle material of an environmentally-friendly gas circuit breaker on the basis of a combination-weighted multi-parameter dynamic decision factor, which is provided by the present invention. A method for evaluating a nozzle material adopts the above arcing test apparatus for evaluating, and specifically includes:

S101: outputting a current signal.

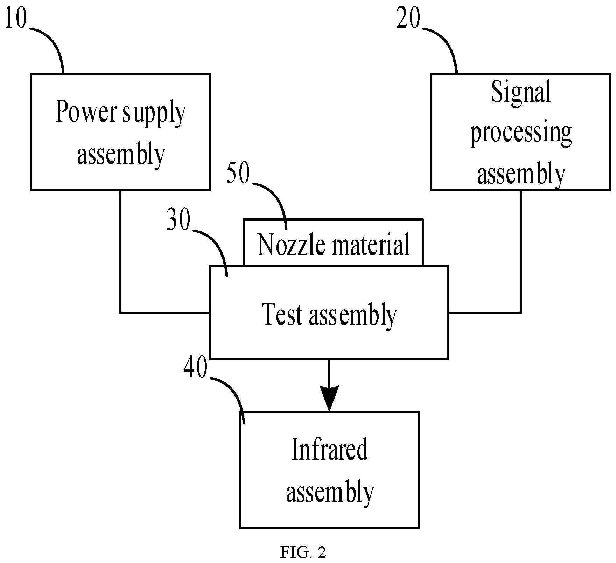

For example, in conjunction with referring to FIG. 2 which is a schematic structure diagram of an example of an arcing test apparatus provided by the present invention, an arcing test is carried out on the nozzle material of the environmentally-friendly gas circuit breaker through adopting the arcing test apparatus, and current data, voltage data, air pressure data and temperature data of the nozzle material in a formation process for an electric arc are obtained. Specifically, a power supply assembly 10 is electrically connected with a test assembly 30 through a cable, the nozzle material 50 is arranged on the test assembly 30 in a sleeved manner, and a current signal is conveyed to the nozzle material 50 through the power supply assembly 10.

S102: carrying out the arcing test on the nozzle material through the current signal to form the electric arc.

For example, continuing to refer to FIG. 2, the power supply assembly 10 is electrically connected with the test assembly 40, the test assembly 40 receives the current signal provided by the power supply assembly 10, and carries out the arcing test on the nozzle material 50 according to the influence generated by the current signal on the test assembly 40 to generate the electric arc on the nozzle material 50.

S103: receiving and processing a sensing electric signal of the nozzle material, and obtaining the current data, the voltage data, and the air pressure data, where the sensing electric signal includes a current-sensing electric signal and an air pressure-sensing electric signal.

For example, in conjunction with referring to FIG. 2, the signal processing assembly 20 first receives a current-sensing electric signal, a voltage-sensing electric signal, and an air pressure-sensing electric signal of the nozzle material 50 when the electric arc burns on the surface of the nozzle material 50, and through processing and analyzing these signals, the current data, the voltage data, and the air pressure data in a test cavity body in the arcing test process are obtained.

S104: detecting temperature data of the nozzle material.

For example, in conjunction with referring to FIG. 2, the infrared assembly 30 is connected with the nozzle material 50 through wireless communication, an infrared signal of the nozzle material 50 in the arcing test process is detected in real time in a manner of non-contact measurement, and temperature data of the nozzle material 50 is obtained through the signal.

S105: evaluating the nozzle material through the current data, the voltage data, the air pressure data, and the temperature data of the nozzle material in the formation process for the electric arc under the actions of different output currents.

For example, a standard voltage parameter, a standard current parameter, a standard air pressure parameter, and a standard temperature parameter are determined according to the current data, the voltage data, the air pressure data, and the temperature data of the nozzle material 50 under the actions of different output currents; a variable voltage parameter, a variable current parameter, a variable air pressure parameter, and a variable temperature parameter are determined according to the standard voltage parameter, the standard current parameter, the standard air pressure parameter, and the standard temperature parameter; a multi-parameter dynamic decision factor is determined according to the variable voltage parameter, the variable current parameter, the variable air pressure parameter, and the variable temperature parameter; a voltage weight, a current weight, an air pressure weight, and a temperature weight are determined according to the multi-parameter dynamic decision factor; a combination-weighted multi-parameter dynamic decision factor is determined according to the voltage weight, the current weight, the air pressure weight, and the temperature weight; and the nozzle material 50 is evaluated according to comparison conditions between the combination-weighted multi-parameter dynamic decision factor, and a qualified threshold value, a good threshold value, as well as an excellent threshold value, and the performance of the nozzle material 50 is classified into four levels, that is, unqualified, qualified, good, and excellent.

From the above description, it may be known that, the arcing test is carried out on the nozzle material of the environmentally-friendly gas circuit breaker, and dynamic changes of the nozzle material and a surrounding environment of the nozzle material in the arcing process are directly reflected through the temperature data, the current data, the voltage data, and the air pressure data of the nozzle material in the formation process for the electric arc. The performance of the nozzle material can be more comprehensively and accurately evaluated through comprehensively considering weights and dynamic changes of a plurality of parameters such as an air pressure, a current, a voltage, and a temperature.

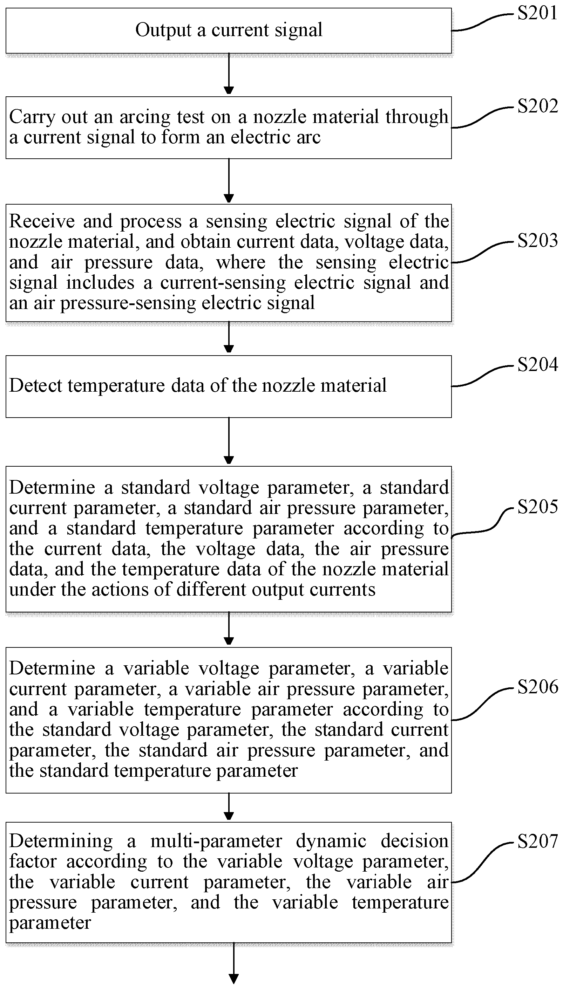

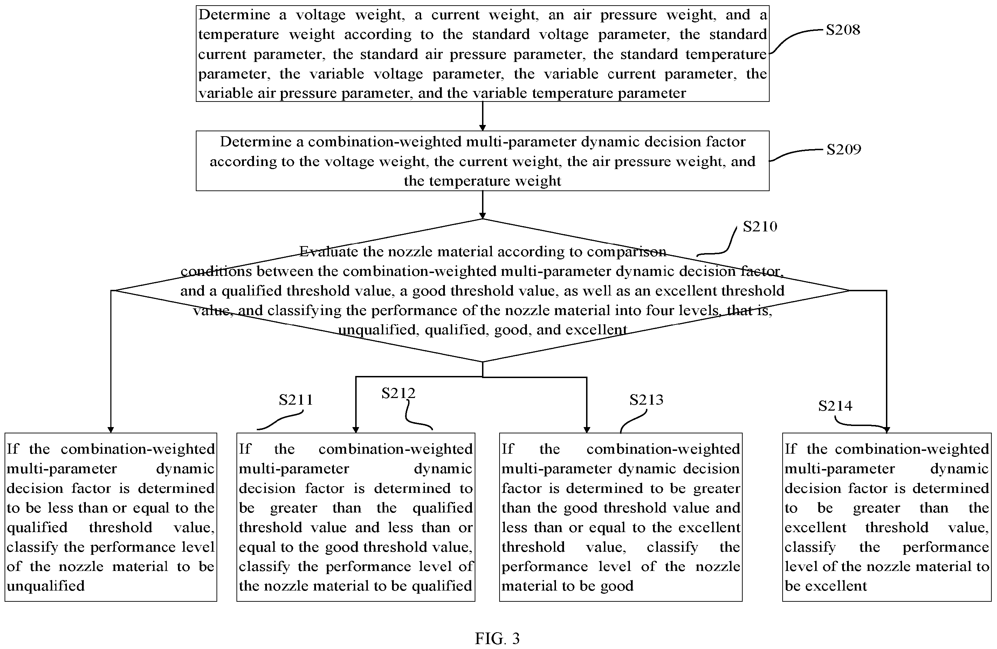

As shown in FIG. 3, FIG. 3 is a schematic flow diagram of another example of a method for evaluating a nozzle material, which is provided by the present invention. A method for evaluating a nozzle material specifically includes:

S201: outputting a current signal.
S202: carrying out the arcing test on the nozzle material through the current signal to form the electric arc.
S203: receiving and processing a sensing electric signal of the nozzle material, and obtaining the current data, the voltage data, and the air pressure data, where the sensing electric signal includes a current-sensing electric signal and an air pressure-sensing electric signal.
S204: detecting temperature data of the nozzle material.

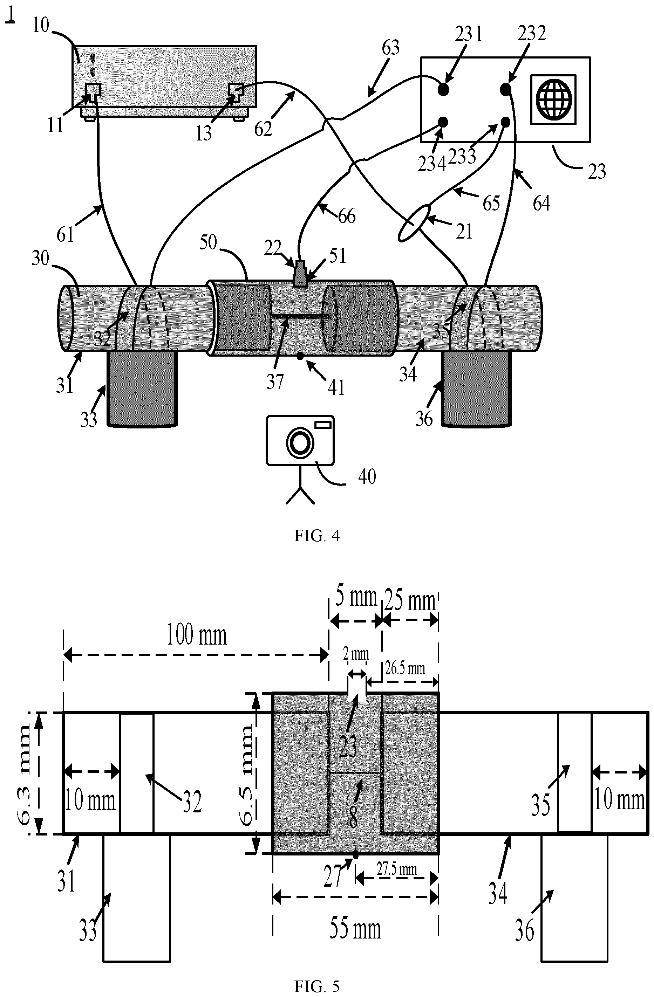

It needs to be noted that, the steps S201 to S204 have been discussed in detail in an implementation scene shown in FIG. 4, and will not be repeated herein.

S205: determining a standard voltage parameter, a standard current parameter, a standard air pressure parameter, and a standard temperature parameter according to the current data, the voltage data, the air pressure data, and the temperature data of the nozzle material under the actions of different output currents.

In conjunction with referring to FIG. 2, current signals with different values are generated through setting an output current of a power supply assembly 10, and the nozzle material is evaluated through the current data, the voltage data, the air pressure data, and the temperature data of the nozzle material 50 in the formation process for the electric arc under the actions of different output currents. Specifically, the output current of the power supply assembly 10 is set to be 1 kA (a first output current), and voltage data from a positive port 231 of a voltage signal and a negative port 232 of the voltage signal are acquired through an integrated processor 23, and recorded as U1; current data from a current signal port 233 is acquired through the integrated processor 23, and recorded as I1; air pressure data from an air pressure signal port 234 is acquired through the integrated processor 23, and recorded as P1; and meanwhile, temperature data from an infrared assembly 40 is acquired, and recorded as T1.

Further, the output current of the power supply assembly 10 is set to be 5 kA, and the voltage data from the positive port 231 of the voltage signal and the negative port 232 of the voltage signal are acquired through the integrated processor 23, and recorded as U2; the current data from the current signal port 233 is acquired through the integrated processor 23, and recorded as I2; the air pressure data from the air pressure signal port 234 is acquired through the integrated processor 23, and recorded as P2; and meanwhile, the temperature data from the infrared assembly 40 is acquired, and recorded as T2.

Further, the output current of the power supply assembly 10 is set to be 10 kA, and the voltage data from the positive port 231 of the voltage signal and the negative port 232 of the voltage signal are acquired through the integrated processor 23, and recorded as U3; the current data from the current signal port 233 is acquired through the integrated processor 23, and recorded as I3; the air pressure data from the air pressure signal port 234 is acquired through the integrated processor 23, and recorded as P3; and meanwhile, the temperature data from the infrared assembly 40 is acquired, and recorded as T3.

Further, the output current of the power supply assembly 10 is set to be 15 kA, and the voltage data from the positive port 231 of the voltage signal and the negative port 232 of the voltage signal are acquired through the integrated processor 23, and recorded as U4; the current data from the current signal port 233 is acquired through the integrated processor 23, and recorded as I4; the air pressure data from the air pressure signal port 234 is acquired through the integrated processor 23, and recorded as P4; and meanwhile, the temperature data from the infrared assembly 40 is acquired, and recorded as T4.

Further, the output current of the power supply assembly 10 is set to be 20 kA, and the voltage data from the positive port 231 of the voltage signal and the negative port 232 of the voltage signal are acquired through the integrated processor 23, and recorded as U5; the current data from the current signal port 233 is acquired through the integrated processor 23, and recorded as I5; the air pressure data from the air pressure signal port 234 is acquired through the integrated processor 23, and recorded as P5; and meanwhile, the temperature data from the infrared assembly 40 is acquired, and recorded as T5.

Further, a standard voltage parameter, a standard current parameter, a standard air pressure parameter, and a standard temperature parameter are determined according to the current data, the voltage data, the air pressure data, and the temperature data of the nozzle material 50, and specifically, the standard voltage parameter is determined according to the following formula:

$$a_i = \frac{0.312 \cdot U_i^2 + 0.186 \cdot U_i + 0.502}{\left(\dfrac{U_1}{1} + \dfrac{U_2}{5} + \dfrac{U_3}{10} + \dfrac{U_4}{15} + \dfrac{U_5}{20}\right)^2} \ ;$$

where $a_i$ is the standard voltage parameter, $U_1$ is the voltage data under a first output current, $U_2$ is the voltage data under a second output current, $U_3$ is the voltage data under a third output current, and $U_4$ is the voltage data under a fourth output current.

The standard current parameter is determined according to the following formula:

$$b_i = \frac{0.312 \cdot I_i^2 + 0.186 \cdot I_i + 0.502}{\left(\dfrac{I_1}{1} + \dfrac{I_2}{5} + \dfrac{I_3}{10} + \dfrac{I_4}{15} + \dfrac{I_5}{20}\right)^2} \ ;$$

where $b_i$ is the standard current parameter, $I_1$ is the current data under the first output current, $I_2$ is the current data under the second output current, $I_3$ is the current data under the
third output current, and $I_4$ is the current data under the fourth output current.

The standard air pressure parameter is determined according to the following formula:

$$c_i = \frac{0.312 \cdot P_i^2 + 0.186 \cdot P_i + 0.502}{\left(\dfrac{P_1}{1} + \dfrac{P_2}{5} + \dfrac{P_3}{10} + \dfrac{P_4}{15} + \dfrac{P_5}{20}\right)^2} \quad ;$$

where $c_i$ is the standard air pressure parameter, $P_i$ is the air pressure data, $P_1$ is the
air pressure data under the first output current, $P_2$ is the air pressure data under the second output current, $P_3$ is the air
pressure data under the third output current, and $P_4$ is the air pressure data under the fourth output current.

The standard temperature parameter is determined according to the following formula:

$$d_i = \frac{0.312 \cdot T_i^2 + 0.186 \cdot T_i + 0.502}{\left(\dfrac{T_1}{1} + \dfrac{T_2}{5} + \dfrac{T_3}{10} + \dfrac{T_4}{15} + \dfrac{T_5}{20}\right)^2} \quad ;$$

where $d_i$ is the standard air pressure parameter, $T_1$ is the temperature data under the
first output current, $T_2$ is the temperature data under the second output current, $T_3$ is the temperature data under the third
output current, and $T_4$ is the temperature data under the fourth output current.

S206: determining a variable voltage parameter, a variable current parameter, a variable air pressure parameter,
and a variable temperature parameter according to the standard voltage parameter, the standard current parameter, the
standard air pressure parameter, and the standard temperature parameter.

For example, the variable voltage parameter is determined according to the following formula:

$$e_1 = 0.312 \cdot \begin{vmatrix} a_1 & 0 & 0 & 0 & 0 \\ a_2 & a_1 & 0 & 0 & 0 \\ a_3 & a_2 & a_1 & 0 & 0 \\ a_4 & a_3 & a_2 & a_1 & 0 \\ a_5 & a_4 & a_3 & a_2 & a_1 \end{vmatrix} + 0.186 \cdot \begin{vmatrix} a_1 & a_2 & a_3 & a_4 & a_5 \\ 0 & a_1 & a_2 & a_3 & a_4 \\ 0 & 0 & a_1 & a_2 & a_3 \\ 0 & 0 & 0 & a_1 & a_2 \\ 0 & 0 & 0 & 0 & a_1 \end{vmatrix} + 0.502 \quad ;$$

where is the standard voltage parameter, $i \in (1, 2, 3, 4, 5)$, and $e_1$ is the variable voltage parameter.

The variable current parameter is determined according to the following formula:

$$e_2 = 0.312 \cdot \overset{a_i}{\begin{vmatrix} b_1 & 0 & 0 & 0 & 0 \\ b_2 & b_1 & 0 & 0 & 0 \\ b_3 & b_2 & b_1 & 0 & 0 \\ b_4 & b_3 & b_2 & b_1 & 0 \\ b_5 & b_4 & b_3 & b_2 & b_1 \end{vmatrix}} + 0.186 \cdot \begin{vmatrix} b_1 & b_2 & b_3 & b_4 & b_5 \\ 0 & b_1 & b_2 & b_3 & b_4 \\ 0 & 0 & b_1 & b_2 & b_3 \\ 0 & 0 & 0 & b_1 & b_2 \\ 0 & 0 & 0 & 0 & b_1 \end{vmatrix} + 0.502 \quad ;$$

where is the standard current parameter, $i \in (1, 2, 3, 4, 5)$, and $e_2$ is the variable current parameter.

The variable air pressure parameter is determined according to the following formula:

$$e_3 = 0.312 \cdot b_i \begin{vmatrix} c_1 & 0 & 0 & 0 & 0 \\ c_2 & c_1 & 0 & 0 & 0 \\ c_3 & c_2 & c_1 & 0 & 0 \\ c_4 & c_3 & c_2 & c_1 & 0 \\ c_5 & c_4 & c_3 & c_2 & c_1 \end{vmatrix} + 0.186 \cdot \begin{vmatrix} c_1 & c_2 & c_3 & c_4 & c_5 \\ 0 & c_1 & c_2 & c_3 & c_4 \\ 0 & 0 & c_1 & c_2 & c_3 \\ 0 & 0 & 0 & c_1 & c_2 \\ 0 & 0 & 0 & 0 & c_1 \end{vmatrix} + 0.502 \quad ;$$

where $c_i$ is the standard air pressure parameter, $i \in (1, 2, 3, 4, 5)$, and $e_3$ is the variable air pressure parameter.

The variable temperature parameter is determined according to the following formula:

$$e_4 = 0.312 \cdot \begin{vmatrix} d_1 & 0 & 0 & 0 & 0 \\ d_2 & d_1 & 0 & 0 & 0 \\ d_3 & d_2 & d_1 & 0 & 0 \\ d_4 & d_3 & d_2 & d_1 & 0 \\ d_5 & d_4 & d_3 & d_2 & d_1 \end{vmatrix} + 0.186 \cdot \begin{vmatrix} d_1 & d_2 & d_3 & d_4 & d_5 \\ 0 & d_1 & d_2 & d_3 & d_4 \\ 0 & 0 & d_1 & d_2 & d_3 \\ 0 & 0 & 0 & d_1 & d_2 \\ 0 & 0 & 0 & 0 & d_1 \end{vmatrix} + 0.502 \quad ;$$

where $e_4$ is the variable temperature parameter, $d_i$ is the standard temperature parameter, and $i \in (1, 2, 3, 4, 5)$.

S207: determining a multi-parameter dynamic decision factor according to the variable voltage parameter, the variable current parameter, the variable air pressure parameter, and the variable temperature parameter.

For example, the multi-parameter dynamic decision factor is determined according to the following formula:

$$\theta = \frac{\sqrt{e_1^2 - 4e_2 e_3}}{e_4} + \frac{\int_0^{e_1} (x^2 \cdot \sin(x) + 0.135 \cdot \cos(x) + \sqrt{e_2^2 - 4 \cdot x e_3 e_4}) dx}{\int_0^{e_2 + e_3} (x^3 \cdot \tan(x) + \frac{e_1}{e_4} \cdot x \cdot \sin(x)) dx} \quad ;$$

where $\theta$ is the multi-parameter dynamic decision factor, $e_1$ is the variable voltage parameter, $e_2$ is the variable current parameter, $e_3$ is the variable air pressure parameter, $e_4$ is the variable temperature parameter, and x is an independent variable in the integration.

S208: determining a voltage weight, a current weight, an air pressure weight, and a temperature weight according to the standard voltage parameter, the standard current parameter, the standard air pressure parameter, the standard temperature parameter, the variable voltage parameter, the variable current parameter, the variable air pressure parameter, and the variable temperature parameter.

For example, the voltage weight is determined according to the following formula:

$$f_1 = e_1 \cdot \frac{\int_0^{e_1} (\sqrt{x^2 - 4e_1}) dx}{\sum_{i=1}^{5} a_i} + 0.205 \cdot e_1^2 \cdot \frac{\sum_{i=1}^{5} a_i}{\prod_{i=1}^{5} a_i} \quad ;$$

where $f_1$ is the voltage weight, $a_i$ is the standard voltage parameter, $i \in (1, 2, 3, 4, 5)$, and $e_1$ is the variable voltage parameter.

The current weight is determined according to the following formula:

$$f_2 = e_2 \cdot \frac{\int_0^{e_2} (\sqrt{x^2 - 4e_2})dx}{\sum_{i=1}^{5} b_i} + 0.205 \cdot e_2^{\ 2} \cdot \frac{\sum_{i=1}^{5} b_i}{\prod_{i=1}^{5} b_i} \ ;$$

where $f_2$ is the current weight, $b_i$ is the standard current parameter, $i \in (1, 2, 3, 4, 5)$, and $e_2$ is the variable current parameter.

The air pressure weight is determined according to the following formula:

$$f_3 = e_3 \cdot \frac{\int_0^{e_3} (\sqrt{x^2 - 4e_3})dx}{\sum_{i=1}^{5} c_i} + 0.205 \cdot e_3^{\ 2} \cdot \frac{\sum_{i=1}^{5} c_i}{\prod_{i=1}^{5} c_i} \ ;$$

where $f_3$ is the air pressure weight, $c_i$ is the standard air pressure parameter, $i \in (1, 2, 3, 4, 5)$, and $e_3$ is the variable air pressure parameter.

The temperature weight is determined according to the following formula:

$$f_4 = e_4 \cdot \frac{\int_0^{e_4} (\sqrt{x^2 - 4e_4})dx}{\sum_{i=1}^{5} d_i} + 0.205 \cdot e_4^{\ 2} \cdot \frac{\sum_{i=1}^{5} d_i}{\prod_{i=1}^{5} d_i} \ ;$$

where $f_4$ is the temperature weight, $d_i$ is the standard temperature parameter, $i \in (1, 2, 3, 4, 5)$, and $e_4$ is the variable temperature parameter.

S209: determining a combination-weighted multi-parameter dynamic decision factor according to the voltage weight, the current weight, the air pressure weight, and the temperature weight.

For example, the combination-weighted multi-parameter dynamic decision factor is determined according to the following formula:

$$\delta = \theta \cdot \left( \frac{f_1 f_2}{2} + \frac{f_1 f_3}{2} + \frac{f_1 f_4}{2} + \frac{f_2 f_3}{2} + \frac{f_3 f_4}{2} \right) \ ;$$

where $f_1$ is the voltage weight, $f_2$ is the current weight, $f_3$ is the air pressure weight, and $f_4$ is the temperature weight.

S210: evaluating the nozzle material according to comparison conditions between the combination-weighted multi-parameter dynamic decision factor, and a qualified threshold value, a good threshold value, as well as an excellent threshold value, and classifying the performance of the nozzle material into four levels, that is, unqualified, qualified, good, and excellent.

For example, the qualified threshold value, the good threshold value, and the excellent threshold value are set to be: $\varepsilon 1 = 0.156$, $\varepsilon 2 = 6.121$, $\varepsilon 3 = 20.156$ respectively. The nozzle material 50 is evaluated according to comparison conditions between the combination-weighted multi-parameter dynamic decision factor and the qualified threshold value, the good threshold value, as well as the excellent threshold value, and the performance of the nozzle material 50 is classified into four levels, that is, unqualified, qualified, good, and excellent.

S211: if the combination-weighted multi-parameter dynamic decision factor is determined to be less than or equal to the qualified threshold value, classifying the performance level of the nozzle material to be unqualified.

For example, if $\delta \le \varepsilon_1$, the performance of the nozzle material being tested at this moment, of the high-voltage circuit breaker is unqualified, and not recommended for use in a high-voltage circuit breaker with any specification.

S212: if the combination-weighted multi-parameter dynamic decision factor is determined to be greater than the qualified threshold value and less than or equal to the good threshold value, classifying the performance level of the nozzle material to be qualified.

For example, if $\varepsilon_1 < \delta \leq \varepsilon_2$, the performance of the nozzle material being tested at this moment, of the high-voltage circuit breaker is qualified, and recommended for use in a high-voltage circuit breaker with a voltage level of 40.5 kV and below.

S213: if the combination-weighted multi-parameter dynamic decision factor is determined to be greater than the good threshold value and less than or equal to the excellent threshold value, classifying the performance level of the nozzle material to be good.

For example, if $\varepsilon_2 < \delta \leq \varepsilon_3$, the performance of the nozzle material being tested at this moment, of the high-voltage circuit breaker is good, and recommended for use in a high-voltage circuit breaker with a voltage level of 126 kV and below.

S214: if the combination-weighted multi-parameter dynamic decision factor is determined to be greater than the excellent threshold value, classifying the performance level of the nozzle material to be excellent.

For example, if $\delta > \varepsilon_3$, the performance of the nozzle material being tested at this moment, of the high-voltage circuit breaker is excellent, and recommended for use in a high-voltage circuit breaker with a voltage level of 252 kV and below.

As shown in FIG. 2, FIG. 2 is a schematic structure diagram of an example of an arcing test apparatus provided by the present invention. An arcing test apparatus 1 is used for obtaining current data, voltage data, air pressure data, and temperature data of a nozzle material 50 in a formation process for an electric arc, and specifically includes: a power supply assembly 10, a signal processing assembly 20, an infrared assembly 30, and a test assembly 40, where the power supply assembly 10 is electrically connected with the test assembly 40, and used for outputting a current signal.

For example, the power supply assembly 10 is a power source of the whole test apparatus, and takes charge of providing a stable and adjustable current signal. Specifically, the power supply assembly 10 is electrically connected with the test assembly 30 through a cable, the nozzle material 50 is arranged on the test assembly 30 in a sleeved manner, and a current signal is conveyed to the nozzle material 50 through the power supply assembly 10, and further, the electric arc is generated according to the influence generated by the current signal on the test assembly 30, and the electric arc burns on the surface of the nozzle material 50, so that an electric arc erosion condition that the nozzle material 50 may encounter in an actual working process of the high-voltage circuit breaker is simulated.

The test assembly 40 is electrically connected with the power supply assembly 10, and used for carrying out an arcing test on the nozzle material through the current signal to form the electric arc.

For example, the test assembly 30 is the core part of the arcing test apparatus to simulate a working environment of the nozzle material 50 of the high-voltage circuit breaker under the action of the electric arc. Specifically, the power supply assembly 10 is electrically connected with the test assembly 40, and the test assembly receives the current signal provided by the power supply assembly 10, and carries out an arcing test on the nozzle material 50 according to the influence generated by the current signal on the test assembly 40.

The signal processing assembly 20 is electrically connected with the test assembly 40, and used for receiving and processing a sensing electric signal of the nozzle material 50, and obtaining the current data, the voltage data, and the air pressure data, where the sensing electric signal includes a current-sensing electric signal and an air pressure-sensing electric signal.

For example, the signal processing assembly 20 is connected with the test assembly, the signal processing assembly 20 first receives a current-sensing electric signal, a voltage-sensing electric signal, and an air pressure-sensing electric signal of the nozzle material 50 when the electric arc burns on the surface of the nozzle material 50, and through processing and analyzing these signals, the current data, the voltage data, and the air pressure data in a test cavity body in the arcing test process are obtained.

The infrared assembly 30 is connected with the nozzle material 50 through wireless communication, and used for detecting temperature data of the nozzle material 50.

For example, the infrared assembly 30 is connected with the nozzle material 50 through wireless communication, an infrared signal of the nozzle material 50 in the arcing test process is detected in real time in a manner of non-contact measurement, and temperature data of the nozzle material 50 is obtained through the signal. Specifically, the infrared assembly 40 includes an infrared thermal imager or an infrared thermometer. The infrared thermal imager can capture a temperature distribution image of the surface of the nozzle material 50, while the infrared thermometer can accurately measure a temperature of a specific point.

From the above description, it may be known that, in the present invention, the power supply assembly is electrically connected with the test assembly, and the stable and controllable current signal is output to the test assembly through the power supply assembly. The test assembly receives the current signal from the power supply assembly, and carries out the arcing test on the nozzle material through the current signal to form the electric arc. The infrared assembly is connected with the nozzle material through wireless communication, and non-contact temperature measurement is realized, so that in the arcing test process, temperature changes of the nozzle material may be monitored in real time without worrying about the influence of a measurement device on a test environment or a result. Meanwhile, the sensing electric signal from the test assembly is received through the signal processing assembly in real time, the sensing electric signal includes the current-sensing electric signal and the air pressure-sensing electric signal, and then the current data, the voltage data, and the air pressure data are extracted out. The nozzle material of the high-voltage circuit breaker is evaluated through the temperature data, the current data, the voltage data, and the air pressure data. The test apparatus of the present invention is simpler and more convenient, the parameters considered are more comprehensive, the data processing manner is more scientific, and the performance of the material in practical application can be more effectively reflected, so that a more scientific basis is provided for selection and optimization for the material.

The temperature data, the current data, the voltage data, and the air pressure data directly reflect the dynamic changes of the nozzle material and a surrounding environment of the nozzle material in the arcing process, and are of great significance for analyzing the electric arc erosion resistance, the electrical insulation performance, the heat dissipation performance, etc. of the nozzle material.

As shown in FIG. 4, FIG. 4 is a schematic structure diagram of another example of an arcing test apparatus provided by the present invention. The test assembly 30 includes a first electrode 31, a second electrode 34, and an arc striking metal wire 37, where the first electrode 31 and the second electrode 34 are connected through the arc striking metal wire 37, and the first electrode 31 and the second electrode 34 are used for receiving a current signal, and providing the current signal to the arc striking metal wire 37.

A nozzle material 50 is arranged at a connection section of the arc striking metal wire 37 in a sleeved manner, one end of the nozzle material 50 is arranged on an end part of the first electrode 31 in a sleeved manner, the other end of the nozzle material 50 is arranged on an end part of the second electrode 34 in a sleeved manner, and a cavity body is formed, and used for accommodating the arc striking metal wire, and the arc striking metal wire is used for carrying out an arcing test on the nozzle material 50 through the current signal to form an electric arc.

For example, in conjunction with referring to FIG. 5, FIG. 5 is an explanatory diagram of key dimensions of the arcing test apparatus provided by the present invention. The first electrode 31 and the second electrode 34 are connected through the arc striking metal wire 37, and the first electrode 31 and the second electrode 34 are used for receiving a current signal, and providing the current signal to the arc striking metal wire 37. A nozzle material 50 is arranged at a connection section of the arc striking metal wire 37 in a sleeved manner, one end of the nozzle material 50 is arranged on an end part of the first electrode 31 in a sleeved manner, the other end of the nozzle material 50 is arranged on an end part of the second electrode 34 in a sleeved manner, the first electrode 31 and the second electrode 34 which are sleeved with the nozzle material 50 are placed in an aligned manner along the horizontal axis, and a distance between the first electrode 31 and the second electrode 34 which are sleeved with the nozzle material 50 is set to be 5 mm. A cavity body is formed among the nozzle material 50, the first electrode 31, and the second electrode 34, and used for accommodating the arc striking metal wire 37, the left side of the arc striking metal wire 37 is connected with the right side of the first electrode 31 sleeved with the nozzle material 50 to be tested, the right side of the arc striking metal wire 37 is connected with the left side of the second electrode 34, and the arc striking metal wire 37 is used for carrying out an arcing test on the nozzle material 50 through the current signal to form an electric arc.

Continuing to refer to FIG. 2, the test assembly 30 further includes a first fixing clamp 32, a first insulation seat 33, a second fastening clamp, and a second insulation seat 36; and the first electrode 31 is connected with the first insulation seat 33 in a fastening manner and through the first fixing clamp 32, the first fixing clamp 32 is used for fixing the first electrode 31, and the first insulation seat 33 is used for supporting the fixed first electrode 31.

The second electrode 34 is connected with the second insulation seat 36 in a fastening manner and through the second fixing clamp, the second fixing clamp is used for fixing the second electrode 34, and the second insulation seat 36 is used for supporting the fixed second electrode 34.

For example, in conjunction with referring to FIG. 5, a current positive port 11 of the power supply assembly 10 is connected with the first fixing clamp 32 through a first cable 61, and a current negative port 12 of the power supply assembly 10 is connected with the second fixing clamp 33 through a second cable 62, so that electric connection between the power supply assembly 10 and the test assembly 30 is realized. The first electrode 31 is placed on a first insulation support seat, and fixed by means of a first conductive fixing clamp on the first insulation support seat, a distance between the first fixing clamp 32 and the left end of the first electrode 31 is set to be 10 mm, the second electrode 34 is placed on a second insulation support seat, and fixed by means of the second fixing clamp on the second insulation support seat, and a distance between the second fixing clamp and the right end of the second electrode 34 is set to be 10 mm.

When the nozzle material 50 is sleeved, the nozzle material 50 is sleeved in from the right side of the first electrode 31, and moved to make contact with the right side of the first insulation support seat, further, the nozzle material 50 is moved to be above the arc striking metal wire 37, and a distance between the right end of the nozzle material 50 and the left end of the second electrode 34 is set to be 25 mm.

Continuing to refer to FIG. 2, the signal processing assembly 20 includes a current mutual-induction coil 21, an air pressure sensor 22, and an integrated processor 23, and the first electrode 31 is electrically connected with a positive pole of the power supply assembly 10; and the second electrode 34 is electrically connected with a negative pole of the power supply assembly 10, and the current mutual-induction coil 21 is arranged on a connection wire, and used for detecting a current-sensing electric signal of the nozzle material 50.

A penetrating hole 51 is formed in the nozzle material 50 on a side wall of the cavity body, and used for accommodating an air pressure sensor 22, and the air pressure sensor 22 is used for detecting an air pressure-sensing electric signal of the nozzle material 50.

The integrated processor 23 is electrically connected with the test assembly 30, and used for receiving and processing a sensing electric signal of the nozzle material 50, and obtaining current data, voltage data, and air pressure data, where the sensing electric signal includes the current-sensing electric signal and the air pressure-sensing electric signal.

For example, the signal processing assembly 20 includes a current mutual-induction coil 21, an air pressure sensor 22, and an integrated processor 23, and the first electrode 31 is electrically connected with a positive pole of the power supply assembly 10 through a first cable 61; the second electrode 34 is electrically connected with a negative pole of the power supply assembly 10 through a second cable 62, the second cable 62 is sleeved with the high-frequency current mutual-induction coil 21, and a current-sensing electric signal of the nozzle material 50 when the electric arc burns on the surface of the nozzle material 50 is received through the current mutual-induction coil 21.

A penetrating hole 51 is formed in the nozzle material 50 on a side wall of the cavity body, the air pressure sensor 22 is placed in the penetrating hole 51 in the nozzle material 50, specifically, other sensors may be installed in the penetrating hole 51 of the nozzle material 50 in a manner of threaded connection, press-fitting or adhesion, etc., so that good sealing between the sensors and an inner wall of a nozzle is guaranteed, and influence on the measurement accuracy due to gas leakage is prevented, and an air pressure-sensing electric signal of the nozzle material 50 when the electric arc burns on the surface of the nozzle material 50 is received through air pressure-sensing.

The integrated processor 23 is electrically connected with the test assembly 30, a voltage-sensing electric signal of the nozzle material 50 when the electric arc burns on the surface of the nozzle material 50 is received through the integrated processor 23, and current data, voltage data, and air pressure data in a test cavity body in the arcing test process are obtained through processing and analyzing the current-sensing signal, the voltage-sensing signal and the air pressure-sensing signal.

Continuing to refer to FIG. 4, the integrated processor 23 includes a voltage signal port, a current signal port 233, and an air pressure signal port 234 in an integrated manner, the current mutual-induction coil 21 is electrically connected with the current signal port 233, and the current signal port 233 is used for receiving the current-sensing electric signal, and obtaining the current data through the current-sensing electric signal.

The first fixing clamp 32 is electrically connected with a positive pole of the voltage signal port, the second fixing clamp is electrically connected with a negative pole of the voltage signal port, and the voltage signal port is used for detecting and processing the voltage-sensing electric signal of the nozzle material 50 to obtain the voltage data.

The air pressure signal port 234 is electrically connected with the air pressure sensor 22, and the air pressure sensor 22 is used for receiving and transmitting the air pressure data in the cavity body.

For example, the voltage signal port includes a voltage-signal positive port 231 and a voltage-signal negative port 232, the voltage-signal positive port 231 of the integrated processor 23 is electrically connected with the first fixing clamp 32 through a positive cable 6363 of a voltage probe, and the voltage-signal negative port 232 of the integrated processor 23 is connected with the second fixing clamp through a negative cable 64 of the voltage probe. The voltage-sensing electric signal of the nozzle material 50 is detected and processed through the voltage signal port, and the voltage data is obtained. The high-frequency current mutual-induction coil 21 is connected with the current signal port 233 of the integrated processor 23 by means of a communication cable 65 of the current coil, the current-sensing electric signal is received through the current signal port 233, and the current data is obtained through the current-sensing electric signal. The air pressure sensor 22 is connected with the air pressure signal port 234 of the integrated processor 23 by means of a communication cable 66 of the air pressure sensor, and the air pressure data in the cavity body is received and transmitted through the air pressure sensor 22.

The infrared assembly 40 is aligned with an infrared temperature measurement point 41 of the nozzle material 50, and a horizontal distance between the infrared assembly 40 and the nozzle material 50 is 2000 mm.

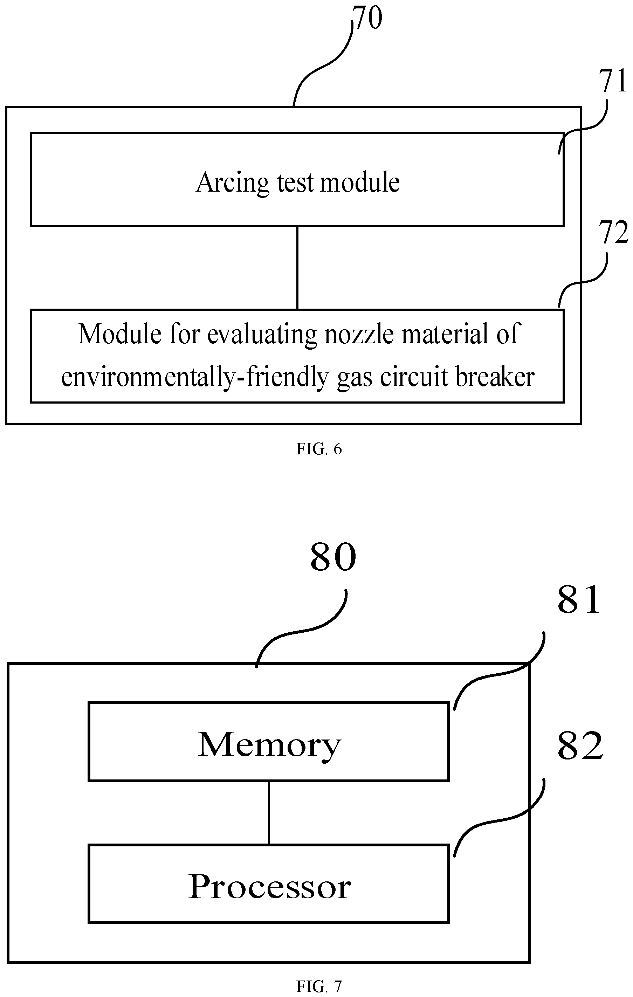

As shown in FIG. 6, FIG. 6 is a schematic structure diagram of an example of a system for evaluating a nozzle material of an environmentally-friendly gas circuit breaker on the basis of a combination-weighted multi-parameter dynamic decision factor, which is provided by the present invention. The system 70 for evaluating the nozzle material of the environmentally-friendly gas circuit breaker on the basis of the combination-weighted multi-parameter dynamic decision factor specifically includes:

an arcing test module 71, used for outputting a current signal; carrying out an arcing test on the nozzle material through the current signal to form an electric arc; receiving and processing a sensing electric signal of the nozzle material, and obtaining current data, voltage data, and air pressure data, where the sensing electric signal includes a current-sensing electric signal and an air pressure-sensing electric signal; and detecting temperature data of the nozzle material.

For example, in the arcing test module 71, an arcing test is carried out on the nozzle material 50 of the environmentally-friendly gas circuit breaker through adopting an arcing test apparatus 1, and current data, voltage data, air pressure data, and temperature data of the nozzle material 50 in a formation process for an electric arc are obtained. Specifically, a current signal is output according to the power supply assembly 10; the test assembly 40 carries out an arcing test on the nozzle material through the current signal to form an electric arc; the signal processing assembly 20 receives and processes a sensing electric signal of the nozzle material, and obtains current data, voltage data, and air pressure data, where the sensing electric signal includes a current-sensing electric signal and an air pressure-sensing electric signal; and the infrared assembly 30 detects temperature data of the nozzle material.

A module 72 for evaluating the nozzle material of the environmentally-friendly gas circuit breaker is used for evaluating the nozzle material of the environmentally-friendly gas circuit breaker through the current data, the voltage data, the air pressure data, and the temperature data of the nozzle material in the formation process for the electric arc under the actions of different output currents.

For example, in the module 72 for evaluating the nozzle material of the environmentally-friendly gas circuit breaker, a standard voltage parameter, a standard current parameter, a standard air pressure parameter, and a standard temperature parameter are determined according to the current data, the voltage data, the air pressure data, and the temperature data of the nozzle material under the actions of different output currents; a variable voltage parameter, a variable current parameter, a variable air pressure parameter, and a variable temperature parameter are determined according to the standard voltage parameter, the standard current parameter, the standard air pressure parameter, and the standard temperature parameter; a multi-parameter dynamic decision factor is determined according to the variable voltage parameter, the variable current parameter, the variable air pressure parameter, and the variable temperature parameter; a voltage weight, a current weight, an air pressure weight, and a temperature weight are determined according to the standard voltage parameter, the standard current parameter, the standard air pressure parameter, the standard temperature parameter, the variable voltage parameter, the variable current parameter, the variable air pressure parameter, and the variable temperature parameter; the combination-weighted multi-parameter dynamic decision factor is determined according to the voltage weight, the current weight, the air pressure weight, and the temperature weight; and the nozzle material is evaluated according to comparison conditions between the combination-weighted multi-parameter dynamic decision factor, and a qualified threshold value, a good threshold value, as well as an excellent threshold value, and the performance of the nozzle material is classified into four levels, that is, unqualified, qualified, good, and excellent.

As shown in FIG. 7, FIG. 7 is a schematic structure diagram of an example of a device provided by the present invention. The device 80 includes a memory 81 and a processor 82. The memory 21 stores a computer program, and the processor 82 executes the computer program to realize the method shown in FIG. 1 and FIG. 3 during working.

Specific technical details with regard to the method for evaluating the nozzle material, which is realized when the above device 20 executes the computer program have been discussed in detail in the above method steps, and therefore will not be repeated herein.

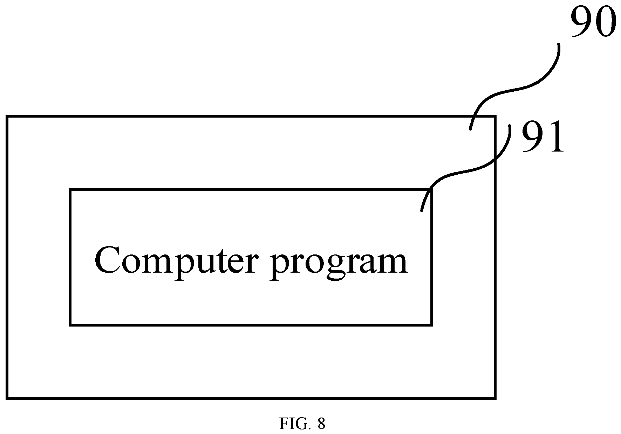

As shown in FIG. 8, FIG. 8 is a schematic structure diagram of an example of a storage medium provided by the present invention. At least one computer program 91 is stored in the storage medium 90, and executed by the processor 82 to realize the method shown in FIG. 1 and FIG. 3, referring to the above for the detailed method, and the detailed method will not be repeated herein. In an example, the medium 90 may be a storage chip, a hard disk, a mobile hard disk, a USB disk, a compact disk, or other readable-writable storage tools, and may also be a server, etc.

The specific examples of the present specification have been described above, and other examples are within the scope of the appended claims. In some cases, the actions or steps recorded in the claims may be executed in a different order from those in the examples and still may realize a desired result. In addition, the processes described in the drawings can realize the desired result not necessarily in the particular order or sequential order shown. In some implementation manners, multitasking and parallel processing are also possible or may be advantageous.

The examples in the specification are described in a progressive manner, identical and similar parts among the examples may refer to each other, and each example focuses on differences from the other examples. Especially, the apparatus example, device example, and non-volatile computer-readable storage medium example are basically similar to the method example, therefore, the descriptions are relatively simple, and referring to the partial description for the method example for relevant information.

The apparatus, device, and non-volatile computer-readable storage medium which are provided by the examples of the present specification correspond to the method, therefore, the apparatus, device, and non-volatile computer-readable storage medium also have similar beneficial technical effects as the corresponding method, and the beneficial technical effects of the method have been described in detail above, therefore, the beneficial technical effects of the corresponding apparatus, device, and non-volatile computer-readable storage medium will not be repeated herein.

The system, apparatus, module, or unit described in the above examples specifically may be realized by a computer chip or an entity, or may be realized by a product with a certain function. A typical realization device is a computer. Specifically, for example, the computer may be a personal computer, a laptop computer, a cellular phone, a camera phone, a smart phone, a personal digital assistant, a media player, a navigation device, an email device, a game console, a tablet computer, a wearable device, or any combination of these devices.

For the convenience of description, while being described, the above apparatus is divided into various units according to functions for separate description. Certainly, when the present specification is implemented, the functions of the units may be realized in one or more pieces of software and/or hardware. Those skilled in the art should understand that, the examples of the present specification may be provided as a method, a system, or a computer program product. Therefore, the examples of the present specification may adopt a form of a completely-hardware example, a completely-software example, or an example combining a software aspect with a hardware aspect. Moreover, the examples of the present specification may adopt the form of a computer program product implemented in one or more computer-usable storage media (including but not limited to a magnetic disk memory, a CD-ROM, an optical memory, etc.) including a computer-usable program code therein.

The present specification is described with reference to the flow diagram and/or the block diagram of the method, device (system), and computer program product according to the examples of the present specification. It should be understood that each flow and/or block in the flow diagram and/or the block diagram, as well as the combination of flows and/or blocks in the flow diagram and/or the block diagram may be realized by computer program instructions. These computer program instructions may be provided to a processor of a general-purpose computer, a special-purpose computer, an embedded processor, or other programmable data processing devices to generate a machine, so that the instructions executed by the processor of the computer or the other programmable data processing devices generate an apparatus for realizing the functions specified in one or more flows of the flow diagram and/or one or more blocks of the block diagram.

These computer program instructions may also be stored in a computer-readable memory that can guide the computer or the other programmable data processing devices to work in a specific manner, so that the instructions stored in the computer-readable memory generate a product including an instruction apparatus, where the instruction apparatus realizes the functions specified in one or more flows of the flow diagram and/or one or more blocks of the block diagram.

These computer program instructions may also be loaded into the computer or the other programmable data processing devices, so that a series of operation steps are executed in the computer or the other programmable data processing devices to generate processing realized by the computer, and then the instructions executed in the computer or the other programmable data processing devices provide steps for realizing the functions specified in one or more flows of the flow diagram and/or one or more blocks of the block diagram.

In a typical configuration, a computing device includes one or more central processing units (CPUs), an input/output interface, a network interface, and a memory.

The memory may be in the form of a volatile memory, a random access memory (RAM) and/or a non-volatile memory, such as a read-only memory (ROM) or a flash RAM, in the computer-readable medium. The memory is an example of the computer-readable medium.

The computer-readable medium includes permanent and non-permanent, removable and non-removable media, and may realize information storage through any method or technology. The information may be a computer-readable instruction, a data structure, a module of a program, or other data. Examples of the storage medium of the computer include but are not limited to a phase change random access memory (PRAM), a static random access memory (SRAM), a dynamic random access memory (DRAM), other types of random access memories (RAM), a read-only memory (ROM), an electrically-erasable programmable read-only memory (EEPROM), a flash memory or other memory technologies, a compact disc read-only memory (CD-ROM), a digital video disk (DVD) or other optical memories, a magnetic-cassette tape, a magnetic-tape magnetic disk memory or other magnetic storage devices, or any other non-transmission medium that may be used for storing information accessible by the computing device. According to the definition in the present specification, the computer-readable medium does not include transitory media such as modulated data signals and carriers.

It further needs to be noted that, the terms 'including', 'containing', or any other variation thereof are intended to encompass non-exclusive inclusion, so that a process, method, commodity, or device that includes a series of elements includes not only those elements, but also other elements not explicitly listed, or elements inherent to such process, method, commodity, or device. In the absence of more limitations, the element defined by the statement 'including one...' does not exclude the existence of other identical elements in the process, method, commodity, or device that includes the element.

The present specification may be described in the general context of computer-executable instructions executed by the computer, such as a program module. Generally, the program module includes routines, programs, objects, assemblies, data structures, etc. that execute specific tasks or realize specific abstract data types. The present specification may also be practiced in distributed computing environments, and in these distributed computing environments, tasks are executed by remote processing devices connected through communication networks. In the distributed computing environments, the program module may be located in local and remote computer storage media that include a storage device.

The examples in the specification are described in a progressive manner, identical and similar parts among the examples may refer to each other, and each example focuses on differences from the other examples. Especially, for the system example, which is basically similar to the method example, the description is relatively simple, and referring to the partial description for the method example for relevant information.

The above disclosure is merely the preferred examples of the present invention, and certainly cannot be used for limiting the scope of rights of the present invention, therefore, equivalent variations made according to the claims of the present invention still fall within the scope of the present invention.

**Claims**

1. A method for evaluating a nozzle material of an environmentally-friendly gas circuit breaker on the basis of a combination-weighted multi-parameter dynamic decision factor, specifically comprising:

   outputting a current signal;
   carrying out an arcing test on the nozzle material through the current signal to form an electric arc;
   receiving and processing a sensing electric signal of the nozzle material, and obtaining current data, voltage data, and air pressure data, wherein the sensing electric signal comprises a current-sensing electric signal and an air pressure-sensing electric signal;
   detecting temperature data of the nozzle material; and
   evaluating the nozzle material of the environmentally-friendly gas circuit breaker through the current data, the voltage data, the air pressure data, and the temperature data of the nozzle material in the formation process for the electric arc under the actions of different output currents.

2. The method for evaluating the nozzle material on the basis of the combination-weighted multi-parameter dynamic decision factor according to claim 1, wherein the evaluating the nozzle material of the environmentally-friendly gas circuit breaker according to the current data, the voltage data, the air pressure data, and the temperature data of the nozzle material in the formation process for the electric arc specifically comprises:

   determining a standard voltage parameter, a standard current parameter, a standard air pressure parameter, and a standard temperature parameter according to the current data, the voltage data, the air pressure data, and the temperature data of the nozzle material under the actions of different output currents;
   determining a variable voltage parameter, a variable current parameter, a variable air pressure parameter, and a variable temperature parameter according to the standard voltage parameter, the standard current parameter, the standard air pressure parameter, and the standard temperature parameter;
   determining a multi-parameter dynamic decision factor according to the variable voltage parameter, the variable current parameter, the variable air pressure parameter, and the variable temperature parameter;
   determining a voltage weight, a current weight, an air pressure weight, and a temperature weight according to the standard voltage parameter, the standard current parameter, the standard air pressure parameter, the standard temperature parameter, the variable voltage parameter, the variable current parameter, the variable air pressure parameter, and the variable temperature parameter;
   determining the combination-weighted multi-parameter dynamic decision factor according to the voltage weight, the current weight, the air pressure weight, and the temperature weight; and
   evaluating the nozzle material according to comparison conditions between the combination-weighted multi-parameter dynamic decision factor, and a qualified threshold value, a good threshold value, as well as an excellent threshold value, and classifying the performance of the nozzle material into four levels, that is, unqualified, qualified, good, and excellent.

3. The method for evaluating the nozzle material on the basis of the combination-weighted multi-parameter dynamic decision factor according to claim 2, wherein the evaluating the nozzle material according to comparison conditions between the combination-weighted multi-parameter dynamic decision factor, and a qualified threshold value, a good threshold value, as well as an excellent threshold value, and classifying the performance of the nozzle material into four levels, that is, unqualified, qualified, good, and excellent specifically comprises:

   if the combination-weighted multi-parameter dynamic decision factor is determined to be less than or equal to the qualified threshold value, classifying the performance level of the nozzle material to be unqualified;
   if the combination-weighted multi-parameter dynamic decision factor is determined to be greater than the qualified threshold value and less than or equal to the good threshold value, classifying the performance level of the nozzle material to be qualified;

if the combination-weighted multi-parameter dynamic decision factor is determined to be greater than the good threshold value and less than or equal to the excellent threshold value, classifying the performance level of the nozzle material to be good; and

if the combination-weighted multi-parameter dynamic decision factor is determined to be greater than the excellent threshold value, classifying the performance level of the nozzle material to be excellent.

4. The method for evaluating the nozzle material according to claim 3, wherein the determining the combination-weighted multi-parameter dynamic decision factor according to the voltage weight, the current weight, the air pressure weight, and the temperature weight specifically comprises:

determining the combination-weighted multi-parameter dynamic decision factor according to

$$\delta = \theta \cdot (\frac{f_1 f_2}{2} + \frac{f_1 f_3}{2} + \frac{f_1 f_4}{2} + \frac{f_2 f_3}{2} + \frac{f_3 f_4}{2})$$, wherein $f_1$ is the voltage weight, $f_2$ is the current weight, $f_3$ is the air pressure weight, and $f_4$ is the temperature weight.

5. An arcing test apparatus, used for obtaining the current data, the voltage data, the air pressure data, and the temperature data of the nozzle material in the formation process for the electric arc according to claim 1, and specifically comprising: a power supply assembly, a signal processing assembly, an infrared assembly, and a test assembly, wherein

the power supply assembly is electrically connected with the test assembly, and used for outputting a current signal;

the test assembly is electrically connected with the power supply assembly, and used for carrying out an arcing test on the nozzle material through the current signal to form an electric arc;

the signal processing assembly is connected with the test assembly, and used for receiving and processing a sensing electric signal of the nozzle material, and obtaining the current data, the voltage data, and the air pressure data, wherein the sensing electric signal comprises a current-sensing electric signal and an air pressure-sensing electric signal; and

the infrared assembly is connected with the nozzle material through wireless communication, and used for detecting temperature data of the nozzle material.

6. The arcing test apparatus according to claim 5, wherein the test assembly comprises a first electrode, a second electrode, and an arc striking metal wire,

the first electrode is connected with the second electrode through the arc striking metal wire, and the first electrode and the second electrode are used for receiving the current signal, and providing the current signal to the arc striking metal wire; and

the nozzle material is arranged at a connection section of the arc striking metal wire in a sleeved manner, one end of the nozzle material is arranged on an end part of the first electrode in a sleeved manner, the other end of the nozzle material is arranged on an end part of the second electrode in a sleeved manner, and a cavity body is formed, and used for accommodating the arc striking metal wire, and the arc striking metal wire is used for carrying out an arcing test on the nozzle material through the current signal to form an electric arc.

7. The arcing test apparatus according to claim 6, wherein the test assembly further comprises a first fixing clamp, a first insulation seat, a second fastening clamp, and a second insulation seat;

the first electrode is connected with the first insulation seat in a fastening manner and through the first fixing clamp, the first fixing clamp is used for fixing the first electrode, and the first insulation seat is used for supporting the fixed first electrode; and

the second electrode is connected with the second insulation seat in a fastening manner and through the second fixing clamp, the second fixing clamp is used for fixing the second electrode, and the second insulation seat is used for supporting the fixed second electrode.

8. The arcing test apparatus according to claim 7, wherein the signal processing assembly comprises a current mutual-induction coil, an air pressure sensor, and an integrated processor, and

the first electrode is electrically connected with a positive pole of the power supply assembly; the second electrode is electrically connected with a negative pole of the power supply assembly, and the current mutual-induction coil is arranged on a connection wire, and used for detecting a current-sensing electric signal of the nozzle material; a penetrating hole is formed in the nozzle material on a side wall of the cavity body, and used for accommodating the air pressure sensor, and the air pressure sensor is used for detecting an air pressure-sensing electric signal of the nozzle material; and

the integrated processor is electrically connected with the test assembly, and used for receiving and processing the sensing electric signal of the nozzle material, and obtaining current data, voltage data, and air pressure data, wherein the sensing electric signal comprises the current-sensing electric signal and the air pressure-sensing electric signal.

9. The arcing test apparatus according to claim 8, wherein the integrated processor comprises a voltage signal port, a current signal port, and an air pressure signal port in an integrated manner,

the current mutual-induction coil is electrically connected with the current signal port, and the current signal port is used for receiving the current-sensing electric signal, and obtaining the current data through the current-sensing electric signal;

the first fixing clamp is electrically connected with a positive pole of the voltage signal port, the second fixing clamp is electrically connected with a negative pole of the voltage signal port, and the voltage signal port is used for detecting and processing a voltage-sensing electric signal of the nozzle material to obtain the voltage data; and

the air pressure signal port is electrically connected with the air pressure sensor, and the air pressure sensor is used for receiving and transmitting the air pressure data in the cavity body.

10. The arcing test apparatus according to claim 9, wherein the infrared assembly receives an infrared signal of the side wall of the cavity body, which is formed by the nozzle material, and obtains the temperature data of the nozzle material through the infrared signal.

11. A system for evaluating a nozzle material of an environmentally-friendly gas circuit breaker on the basis of a combination-weighted multi-parameter dynamic decision factor, specifically comprising:

an arcing test module, used for outputting a current signal; carrying out an arcing test on the nozzle material through the current signal to form an electric arc; receiving and processing a sensing electric signal of the nozzle material, and obtaining current data, voltage data, and air pressure data, wherein the sensing electric signal comprises a current-sensing electric signal and an air pressure-sensing electric signal; and detecting temperature data of the nozzle material; and

a module for evaluating the nozzle material of the environmentally-friendly gas circuit breaker, used for evaluating the nozzle material of the environmentally-friendly gas circuit breaker through the current data, the voltage data, the air pressure data, and the temperature data of the nozzle material in the formation process for the electric arc under the actions of different output currents.

12. A computer-readable storage medium, storing a computer program, wherein when the computer program is executed by a processor, the processor is enabled to execute the steps of the method according to claim 1.

13. A computer device, comprising a memory and a processor, wherein the memory stores a computer program, and when the computer program is executed by the processor, the processor is enabled to execute the steps of the method according to claim 1.

S101

Output a current signal

S102

Carry out an arcing test on a nozzle material through a current signal to form an electric arc

S103

Receive and process a sensing electric signal of the nozzle material, and obtain current data, voltage data, and air pressure data, where the sensing electric signal includes a current-sensing electric signal and an air pressure-sensing electric signal

S104

Detect temperature data of the nozzle material

S105

Evaluate the nozzle material through the current data, the voltage data, the air pressure data, and the temperature data of the nozzle material in the formation process for the electric arc under the actions of different output currents

FIG. 1

FIG. 2

Output a current signal — S201

Carry out an arcing test on a nozzle material through a current signal to form an electric arc — S202

Receive and process a sensing electric signal of the nozzle material, and obtain current data, voltage data, and air pressure data, where the sensing electric signal includes a current-sensing electric signal and an air pressure-sensing electric signal — S203

Detect temperature data of the nozzle material — S204

Determine a standard voltage parameter, a standard current parameter, a standard air pressure parameter, and a standard temperature parameter according to the current data, the voltage data, the air pressure data, and the temperature data of the nozzle material under the actions of different output currents — S205

Determine a variable voltage parameter, a variable current parameter, a variable air pressure parameter, and a variable temperature parameter according to the standard voltage parameter, the standard current parameter, the standard air pressure parameter, and the standard temperature parameter — S206

Determining a multi-parameter dynamic decision factor according to the variable voltage parameter, the variable current parameter, the variable air pressure parameter, and the variable temperature parameter — S207

Determine a voltage weight, a current weight, an air pressure weight, and a temperature weight according to the standard voltage parameter, the standard current parameter, the standard air pressure parameter, the standard temperature parameter, the variable voltage parameter, the variable current parameter, the variable air pressure parameter, and the variable temperature parameter — S208

Determine a combination-weighted multi-parameter dynamic decision factor according to the voltage weight, the current weight, the air pressure weight, and the temperature weight — S209

Evaluate the nozzle material according to comparison conditions between the combination-weighted multi-parameter dynamic decision factor and a qualified threshold value, a good threshold value, as well as an excellent threshold value, and classifying the performance of the nozzle material into four levels, that is, unqualified, qualified, good, and excellent — S210

S211

S212

S213

S214

If the combination-weighted multi-parameter dynamic decision factor is determined to be less than or equal to the qualified threshold value, classify the performance level of the nozzle material to be unqualified

If the combination-weighted multi-parameter dynamic decision factor is determined to be greater than the qualified threshold value and less than or equal to the good threshold value, classify the performance level of the nozzle material to be qualified

If the combination-weighted multi-parameter dynamic decision factor is determined to be greater than the good threshold value and less than or equal to the excellent threshold value, classify the performance level of the nozzle material to be good

If the combination-weighted multi-parameter dynamic decision factor is determined to be greater than the excellent threshold value, classify the performance level of the nozzle material to be excellent

FIG. 3

FIG. 4

FIG. 5

Arcing test module

Module for evaluating nozzle material of
environmentally-friendly gas circuit breaker

FIG. 6

Memory

Processor

FIG. 7

90

91

Computer program

FIG. 8

Europäisches
Patentamt
European
Patent Office
Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 24 20 9172

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 111 718 550 B (YUNNAN POWER GRID CO LTD ELECTRIC POWER RES INST) 20 May 2022 (2022-05-20) * paragraphs [0059] - [0061]; table 3 * ----- | 1-10 | INV. H01H11/00 ADD. H01H33/70 |
| A | JP S53 147192 U (HITACHI CHEMICAL CO., LTD.) 20 November 1978 (1978-11-20) * the whole document * ----- | 1-10 | |
| A | CN 203 929 698 U (HECHI POWER SUPPLY BUREAU GUANGXI POWER GRID CO LTD ET AL.) 5 November 2014 (2014-11-05) * the whole document * ----- | 1-10 | |
| A | CN 110 501 368 A (UNIV WUHAN) 26 November 2019 (2019-11-26) * the whole document * ----- | 1-10 | |

**TECHNICAL FIELDS
SEARCHED (IPC)**

H01H
G01N

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| Munich | 30 June 2025 | Ledoux, Serge |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................................
& : member of the same patent family, corresponding
    document

EPO FORM 1503 03.82 (P04E07)

Europäisches
Patentamt

European
Patent Office

Office européen
des brevets

**INCOMPLETE SEARCH
SHEET C**

Application Number

EP 24 20 9172

Claim(s) completely searchable:
        5-10

Claim(s) searched incompletely:
        1-4

Claim(s) not searched:
        11-13

Reason for the limitation of the search:

1       The application contains a plurality of independent claims which do not comply with Rule 43(2) EPC.

1.1     Device claims 5, 11, 12 and 13 have been drafted as independent device claims.

1.2     Claim 5 and 11 have partially overlapping technical features and relate to more or less to a system/device/apparatus for testing a material with an arcing test. The subject-matter of claims 5 and 11 are therefore not interrelated devices in the sense of Rule 43(2)(a) EPC. They also do not appear to be alternative solutions to a particular problem, since they have common technical features for solving the problem posed.

1.3     Claim 12 relates to a computer-readable storage medium. According to the actual wording of claim 12, the claimed storage medium, merely stores a computer program, wherein when the computer program is executed by a processor, the processor is enabled to perform a method. Claim 12 does not say that the computer program of the claimed storage medium relates to the method according to claim 1. The computer program performing the method steps of claim 1, when executed in a processor, can be stored on another storage medium. The computer program of the claimed storage medium can merely enable/allow the processor to execute the computer program performing the method of claim 1, which is stored on another storage medium.

The same interpretation applies for the computer device of claim 13, which actually comprises a memory corresponding to the claimed storage medium of claim 12. The computer device does not necessarily have in its memory a computer program for the execution of the method of claim 1. This computer program executing the method of claim 1 could be stored on a storage medium external to the claimed computer device.

The subject-matter of claims 12 and 13 is therefore not interrelated with a device or a system performing nozzle material arching test, contrary to the subject-matter of claims 5 and 11.

1.4     Consequently, independent device claims 5, 11, 12 and 13 do not fall within the exception of Rule 43(2) EPC.

2       A complete search cannot be performed for the subject-matter of claims 1 to 4, because the subject-matter of these claims is not clear (Art. 84 EPC).

Europäisches
Patentamt

European
Patent Office

Office européen
des brevets

**INCOMPLETE SEARCH
SHEET C**

Application Number

EP 24 20 9172

2.1    Claim 1 defines a method for evaluating a nozzle material, which uses a combination-weighted multi-parameter dynamic decision factor. This factor is an unusual parameter which requires a clear definition, which is a priori not part of the common general knowledge of the person skilled in the art, in order to allow the skilled person to carry out the claimed method (see Guidelines F-IV, 4.11.1).

The subject-matter of claim 1 is therefore unclear (Article 84 EPC).

2.2    For evaluating a nozzle material, it is essential to clearly define the combination-weighted multi-parameter dynamic decision factor in claim 1. It is clear from the description in paragraphs [0074] and [0082] to [0119] and figure 3 that:

a combination-weighted multi-parameter dynamic decision factor is determined according to a voltage weight, a current weight, an air pressure weight, and a temperature weight,
the voltage weight, the current weight, the air pressure weight, and the temperature weight are determined according to a multi-parameter dynamic decision factor,
the multi-parameter dynamic decision factor is determined according to a variable voltage parameter, a variable current parameter, a variable air pressure parameter, and a variable temperature parameter,
the variable voltage parameter, the variable current parameter, the variable air pressure parameter, and the variable temperature parameter are determined according to a standard voltage parameter, a standard current parameter, a standard air pressure parameter, and a standard temperature parameter, and
the standard voltage parameter, the standard current parameter, the standard air pressure parameter, and the standard temperature parameter are determined according to the current data, the voltage data, the air pressure data, and the temperature data of the nozzle material under the actions of different output currents obtained during an arching test.

2.3    Since independent claim 1 does not contain all these features and their respective definition as in paragraphs [0088] to [0119], claim 1 does not meet the requirement following from Article 84 EPC, taken in combination with Rule 43(1) and (3) EPC, that any independent claim must contain all the technical features essential to the definition of the invention.

2.4    The same objection applies to dependent claims 2 to 4 which do not provide all the essential features required for the proper definition of the claimed method.

3    The Applicant did not respond to the invitation under Rule 62a(1) and 63(1) EPC dated 11-04-2025. The search is carried out on the basis of the detailed method exposed in the paragraphs [0088] to [0119] for the subject-matter of method claims 1 to 4 (Rule 63(3) EPC).

The search is further carried out on the subject-matter of device claims 5 to 10. The subject-matter of claims 11 to 13 is disregarded (Rule 62a(2) EPC).

## ANNEX TO THE EUROPEAN SEARCH REPORT
## ON EUROPEAN PATENT APPLICATION NO.

EP 24 20 9172

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2025

| Patent document cited in search report | | Publication date | Patent family member(s) | Publication date |
|---|---|---|---|---|
| CN 111718550 | B | 20-05-2022 | NONE | |
| JP S53147192 | U | 20-11-1978 | NONE | |
| CN 203929698 | U | 05-11-2014 | NONE | |
| CN 110501368 | A | 26-11-2019 | NONE | |

EPO FORM P0459

For more details about this annex : see Official Journal of the European Patent Office, No. 12/82